# EUROPEAN PATENT APPLICATION

(11) **EP 3 875 464 A1**
(43) Date of publication of application: **08.09.2021**
(21) Application number: 20161579.6
(22) Date of filing: 06.03.2020
(51) Int. Cl.: C07J 3/00

(54) **PROCESS FOR THE PURIFICATION OF ETIOCHOLENIC ACID**

(71) Applicant: F.I.S.- Fabbrica Italiana Sintetici S.p.A., 36075 Montecchio Maggiore (VI) (IT)
(72) Inventor: De Filippo, Christian Corrado, 36075 Montecchio Maggiore (IT); Lora, Giovanni, 36075 Montecchio Maggiore (IT); Faccin, Nicola, 36075 Montecchio Maggiore (IT); Zanini, Davide, 36075 Montecchio Maggiore (IT); Peretto, Daniele, 36075 Montecchio Maggiore (IT)
(74) Representative: Leganza, Alessandro

(57) **Abstract**

The present invention relates to a process for the purification of ethiocholenic acid through an hemi-solvate form, wherein said hemi-solvate form is the ethiocholenic acid toluene hemi-solvate of formula (II).

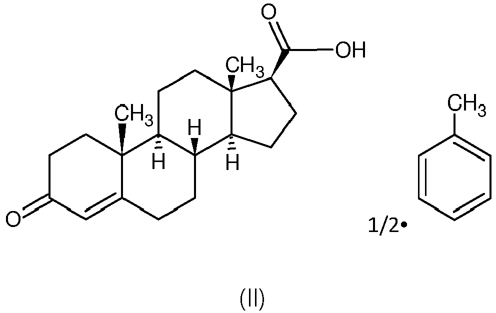

## Description

### Technical field of the invention

The present invention relates to a process for the purification of etiocholenic acid, i.e. a process for preparing purified etiocholenic acid through an hemi-solvate form. Moreover, the present invention relates also to the form itself.

### Background of the invention

WO 2006/002966 discloses the synthesis of etiocholenic acid, particularly example 2 discloses the purification of etiocholenic acid by crystalization with a mixture of ethanol and water resulting to a 110 g of purified product (starting from 170 g of crude etiocholenic acid) and HPLC 99% w/w, 99% area (molar yield = 64.70%).

A.P.J. Brunskill et al. (Acta Crystallographica, Section C: Crystal Structure Communications, 1997, 53, 903-906), discloses a single cell crystal structure of a polymorphic form of etiocholenic acid.

### Summary of the invention

Therefore, the problem addressed by the present invention is to provide an improved process for the preparation of etiocholenic acid in order to achieve increased yields and purity, as well as an enhanced impurity profile of the final product, compared to those obtainable through the methods known in the prior art.

The above-mentioned problems are solved by an hemi-solvate intermediate which allows to achieve increased yield and to enhance purity, including the improvement of the impurity profile.

Further characteristics and advantages of the process according to the invention will become apparent from the below-reported description of preferred embodiments.

### Brief description of the drawings

Figure 1 shows the XPRD diffractogram of the solid form of etiocholenic acid toluene hemi-solvate of formula (II).
Figure 2 shows the XPRD diffractogram of the solid form of etiocholenic acid of formula (I).
Figures 3 and 4 show the DSC for etiocholenic acid toluene hemi-solvate samples.
Figure 5 shows the simulated XPRD pattern of the disclosed solid form of etiocholenic acid as disclosed by A.P.J. Brunskill et al. (Acta Crystallographica, Section C: Crystal Structure Communications, 1997, 53, 903-906).
Figures 6 shows the ¹H-NMR spectrum of etiocholenic acid toluene hemi-solvate of formula (II) (integrated and not-integrated toluene peak).

### Detailed description of the invention

According to a first aspect, the present invention relates to a process for purifying etiocholenic acid, i.e. for preparing purified etiocholenic acid of formula (I): 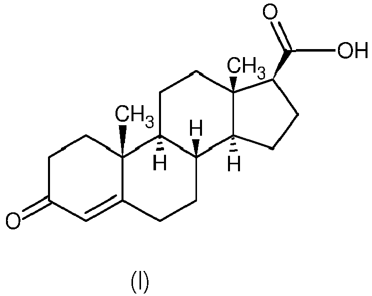 comprising the following steps:
a) preparing etiocholenic acid toluene hemi-solvate of formula (II) 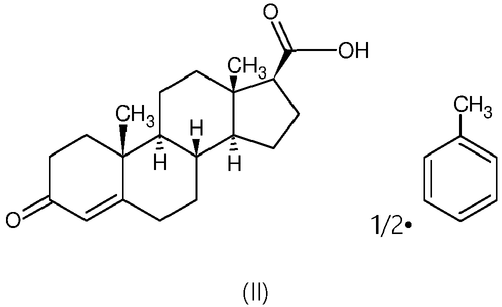 by treating crude etiocholenic acid with a solvent mixture comprising toluene;
b) converting the etiocholenic acid toluene hemi-solvate prepared in step a) to purified etiocholenic acid of formula (I).

According to a preferred embodiment, the etiocholenic acid toluene hemi-solvate of formula (II) has a characteristic X-ray powder diffraction pattern with characteristic peaks expressed in 2-Theta values (2θ) at 16.6 ± 0.2 and/or at 14.4 ± 0.2 and/or at 8.2 ± 0.2, preferably at 16.6 ± 0.2.

According to another preferred embodiment, the etiocholenic acid toluene hemi-solvate has a DSC onset peak at a temperature comprised among 110 °C and 120 °C and/or a peak at a temperature among 120 °C and 130 °C.

As for the toluene contained in the solvent mixtures of step a), the solvent mixture preferably comprises at least 30 % by volume of toluene, more preferably from 30 % to 60 % by volume of toluene, even more preferably from 40 to 50 % by volume of toluene.

As intended herein, the term % by volumes means volume of solvent per volume of solvent mixture, thus, for example, 50% by volume is 500 ml per 1 Liter of solvent mixture.

The solvent mixture, in addition to toluene, can comprise one or more additional solvents. According to a preferred aspect, the solvent mixture further comprises additional solvent selected from acetone and water. Preferably, the solvent mixture comprises toluene, acetone and water.

According to a preferred aspect, the solvent mixture consists of a mixture of toluene, acetone and water.

According to a preferred aspect, both in case the solvent mixture comprises toluene, acetone and water and in case the solvent mixture consists of toluene, acetone and water, the volume ratio between toluene : acetone : water is from 1.75 : 0.25 : 1.25 to 3.25 :1.75 : 2.75, preferably from 2 : 0.5 :1.5 to 3 : 1.5 : 2.5, even more preferably from 2.25 : 0.75 : 1.75 to 2.75 : 1.25 : 2.25. According to a particularly preferred aspect, the volume ratio between toluene: acetone : water is 2.45 : 1 : 2.

According to a preferred aspect, the volumes of the solvent mixture per mass unit used in the step a) in the preparation of etiocholenic acid toluene hemi-solvate (II) are from 0.5 to 20, preferably from 1 to 10 volumes per mass unit, more preferably from 1 to 5 volumes per mass unit, even more preferably 2 volumes.

Volumes means volume of solvent per mass unit of product, thus, for example, 1 volume is 1 Liter per 1 Kilo, or 1 mL for 1 gram, or 1 microliter per 1 milligram. Thus, 10 volumes means, for example, 10 liters per 1 Kilogram of substance.

As for step b) of conversion, it can be carried out by solvent suspension of the solid or by heating the solid. According to a preferred aspect, as for step b) of conversion, the etiocholenic acid toluene hemi-solvate is suspended in a solvent, preferably acetone. Preferably, the etiocholenic acid toluene hemi-solvate is suspended in a solvent, preferably in acetone. According to a preferred embodiment, the suspension in acetone is carried out for a time from 0.5 hrs to 40 hrs, more preferably from 0.5 hrs to 20 hrs, even more preferably from 0.5 hrs to 5 hrs. After that time under stirring etiocholenic acid is produced, the suspension is filtered and the purified etiocholenic acid is collected as wet solid over the filter. The product is then dried under vacuum. According to a preferred aspect, the suspension of etiocholenic acid is filtered.

According to a second aspect, the present invention relates to etiocholenic acid toluene hemi-solvate of formula (II):

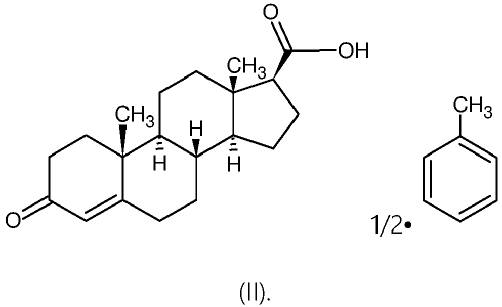

The etiocholenic acid toluene hemi-solvate of formula (II) is characterized by the features as defined above.

According to a third aspect, the present invention relates to the use of etiocholenic acid toluene hemi-solvate of formula (II): 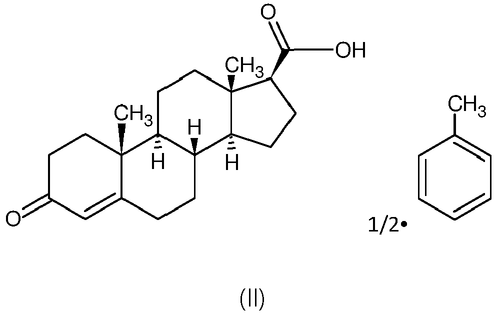 for preparing etiocholenic acid. Preferably, the etiocholenic acid toluene hemi-solvate is suspended in acetone for a time from 0.5 hrs to 40 hrs, preferably from 0.5 hrs to 20 hrs, more preferably from 0.5 hrs to 5 hrs, and the suspension of etiocholenic acid is then filtered.

### EXPERIMENTAL SECTION

All the raw materials are commercially available, for example by Sigma-Aldrich.

### Example 1 - preparation process of etiocholenic acid toluene hemi-solvate

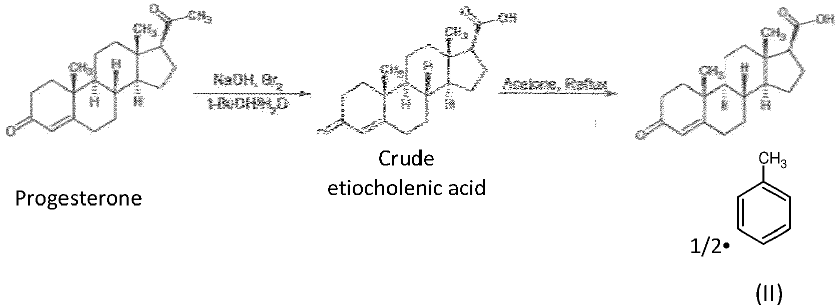

Sodium hydroxide 30 %W 346 ml and purified water 410 ml were charged under nitrogen at 22/25 °C, and the solution was cooled at 3°C. Bromine 260 g was charged in 1h under nitrogen atmosphere keeping the internal temperature at 3 °C. At the end of addition, the solution was kept at 3 °C for 50 min. Terbutanol 950 ml and progesterone 100 g was charged in a second reactor under nitrogen atmosphere. Subsequently, sodium hydroxide 30 %W 313 ml and purified water 337 ml were added. The mixture was heated at 36 °C for 12 minutes to complete the solution, and then the mixture was cooled at 0 °C. The solution of bromine/sodium hydroxide was slowly charged in a period of 65 min on the progesterone suspension keeping the temperature at 0 °C. At the end of the addition, the mixture was kept at 0 °C for 8 hours under vigorous stirring (400 rpm). Then, hydrochloric acid sol. 32%W 410 ml was charged to correct the pH at 2.5 at a Tₘₐₓ< 1 °C, thus obtaining a yellow-orange suspension. The yellow-orange suspension was charged by keeping the internal temperature below 0 °C. 10 g sodium metabisulphite in 160 ml of purified water was added to the yellow suspension by keeping the internal temperature below 0 °C, thus obtaining a pH of 1,5. The suspension obtained was kept at 0 °C for 30 min and then heated at 33 °C and 100 ml terbutanol was charged under stirring at 33 °C for 7 min so that the phases were splitted. The reaction mixture (9.1 V) was distilled at a pressure < 16 mbar and Tjacket max of 43 °C. A solution of crude etiocholenic acid in tert-butanol was thus obtained. Toluene 560 ml was charged and the reaction mixture 3.8 V was distilled at reduced pressure at a pressure < 16 mbar and T jacket max of 43 °C. Toluene 245 ml, acetone 100 ml and 200 ml purified water were charged at 21 °C, controlling the pH under a value of pH= 3. The mixture was heated at 76 °C (reflux), keeping the temperature for 27 min, then the suspension was cooled down in 1 h at 1 °C. The suspension was filtered and washed with an iced-cold mixture of toluene 90 ml and acetone 10 ml to obtain etiocholenic acid wet 110 g, and the solid was dried at 57 °C and a pressure <20 mbar for 10 hours to obtain 76 g of etiocholenic acid toluene hemi-solvate (weight yield 76%). Purity HPLC 99.26% A/A.

### Example 2 - conversion to purified etiocholenic acid (step 6)

The etiocholenic acid toluene hemi-solvate of formula (II) 55 (g) was suspended in acetone (3 V), the suspension was heated at reflux for 0.5 hrs, keeping the suspension at -1°C for 30 min. The suspension was filtered and the panel washed with acetone (1 V) and the solid dried (T=57 °C and p<20 mbar for 10 hrs). 45,5 (g) of purified etiocholenic acid of formula (I) was obtained (molar yield: 94.8%). Purity HPLC 99.4% A/A.

### Example 3 - simulation of XPRD from the prior art

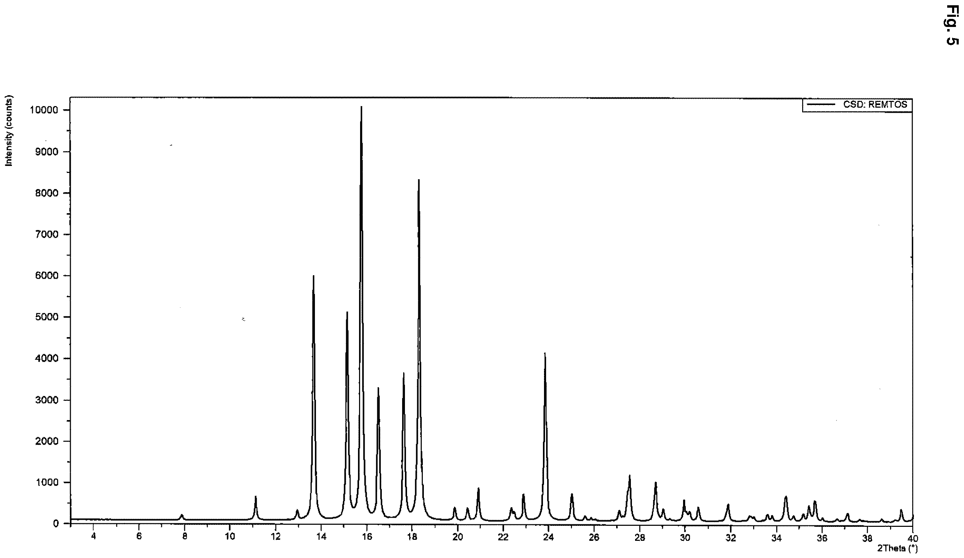

Starting from the X-ray single crystal structure, and related cell structure, disclosed in A.P.J. Brunskill et al. (Acta Crystallographica, Section C: Crystal Structure Communications, 1997, 53, 903-906), the XPRD pattern was simulated (see figure 5). The corresponding data are also reported in the following table 1.

**Table 1**

| Pos. [°2θ] | d-Spacing [Å] | Rel. int. [%] |
|---|---|---|
| 7.87 | 11.23 | 1 |
| 11.12 | 7.95 | 6 |
| 12.94 | 6.83 | 2 |
| 13.68 | 6.47 | 59 |
| 15.16 | 5.84 | 50 |
| 15.78 | 5.61 | 100 |
| 16.52 | 5.36 | 31 |
| 17.64 | 5.03 | 35 |
| 18.32 | 4.84 | 83 |
| 19.87 | 4.47 | 3 |
| 20.43 | 4.34 | 3 |
| 20.91 | 4.25 | 8 |
| 22.35 | 3.97 | 3 |
| 22.49 | 3.95 | 2 |
| 22.89 | 3.88 | 6 |
| 23.85 | 3.73 | 41 |
| 25.01 | 3.56 | 7 |
| 25.59 | 3.48 | 1 |

| Pos. [° 2θ] | d-Spacing [Å] | Rel. int. [%] |
|---|---|---|
| 25.86 | 3.44 | 1 |
| 26.06 | 3.42 | 0 |
| 27.09 | 3.29 | 2 |
| 27.45 | 3.25 | 6 |
| 27.56 | 3.23 | 11 |
| 28.70 | 3.11 | 9 |
| 29.03 | 3.07 | 3 |
| 29.34 | 3.04 | 0 |
| 29.93 | 2.98 | 5 |
| 30.19 | 2.96 | 2 |
| 30.57 | 2.92 | 3 |
| 31.87 | 2.81 | 4 |
| 32.81 | 2.73 | 1 |
| 33.02 | 2.71 | 1 |
| 33.59 | 2.67 | 2 |
| 33.81 | 2.65 | 1 |
| 34.42 | 2.60 | 6 |
| 34.74 | 2.58 | 1 |
| 35.16 | 2.55 | 2 |
| 35.42 | 2.53 | 4 |
| 35.70 | 2.51 | 5 |
| 36.02 | 2.49 | 1 |
| 36.66 | 2.45 | 1 |
| 37.12 | 2.42 | 2 |
| 37.66 | 2.39 | 1 |
| 38.62 | 2.33 | 1 |
| 39.48 | 2.28 | 3 |

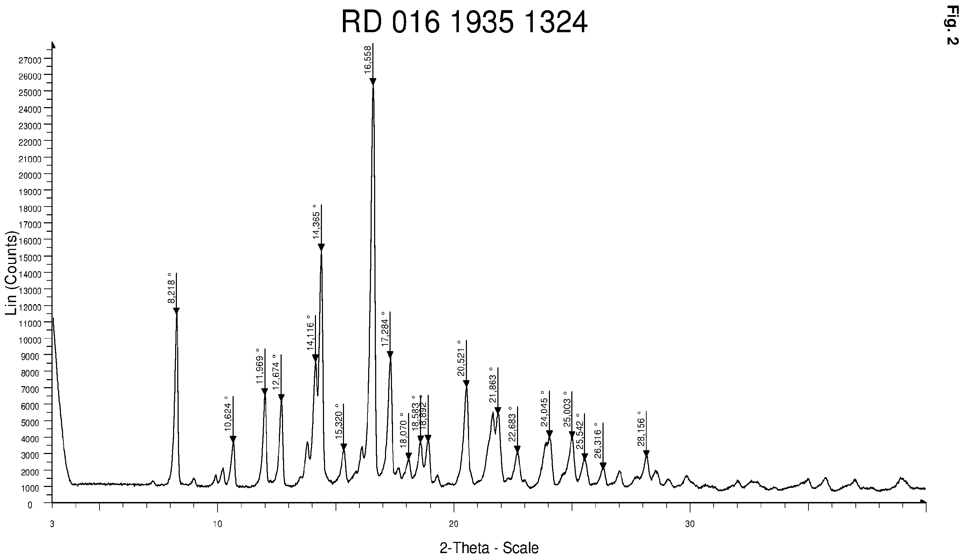

From the comparison of the simulated XPRD spectrum of the prior art document (see figure 5) and the experimental diffractogram of purified etiocholenic acid (see figure 2), as prepared in Example 2, it was possible to confirm that the crystal structure of the purified etiocholenic acid of formula (I) prepared in Example 2 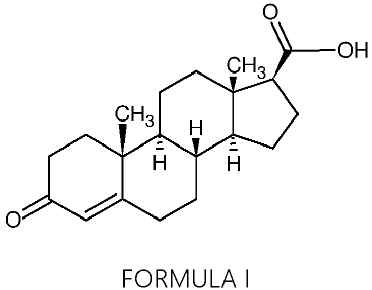 was already known by A.P.J. Brunskill et al. (Acta Crystallographica, Section C: Crystal Structure Communications, 1997, 53, 903-906.

### Example 4 - ¹H-NMR

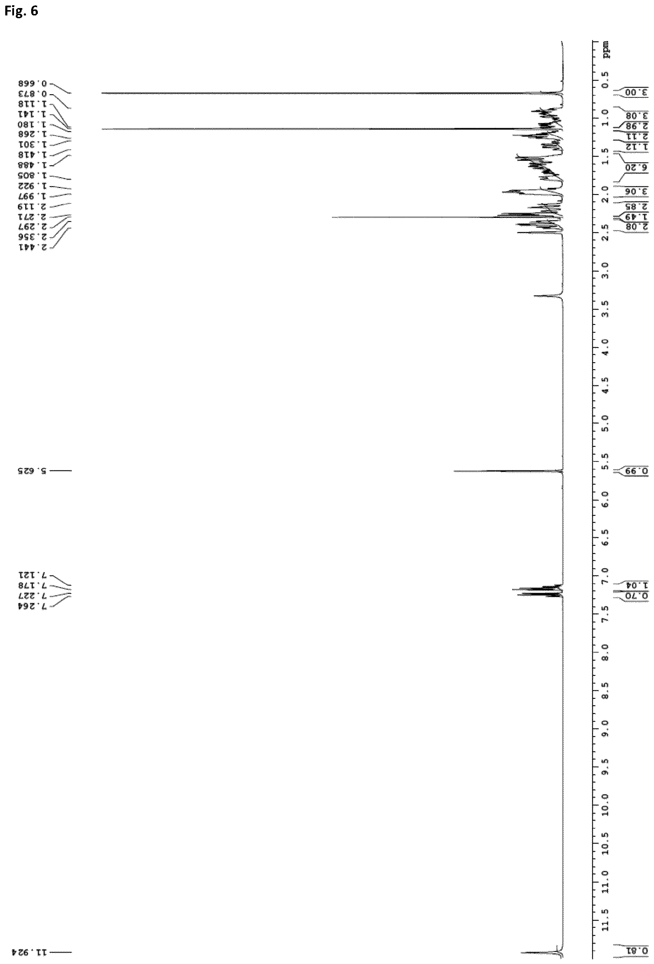

The definition of the stoichiometry of the etiocholenic acid toluene hemi-solvate of formula (II) is clearly showed in the ¹H-NMR spectrum of figure 6, wherein the toluene peak (corresponding to methyl protons) was integrated.

### Example 5 - DSC Analysis

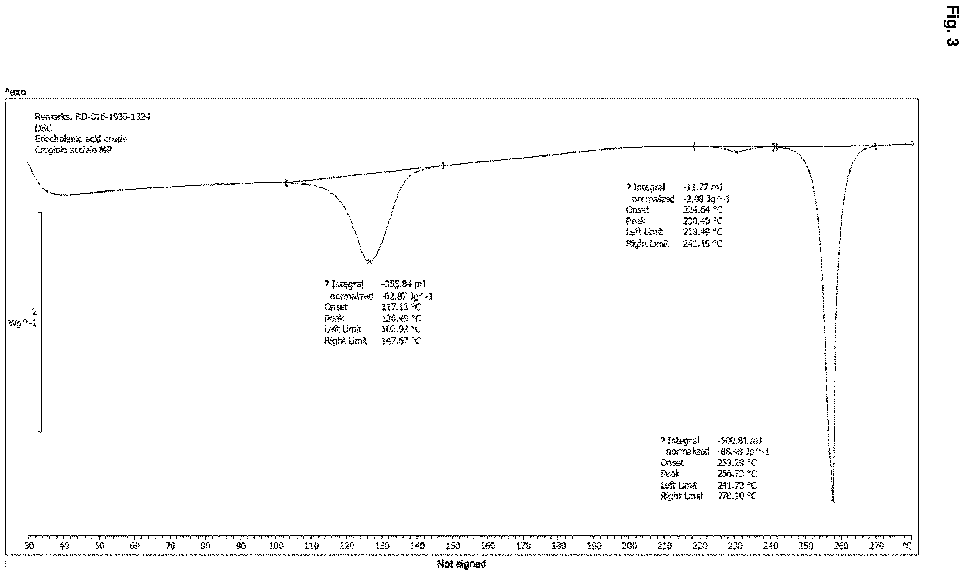

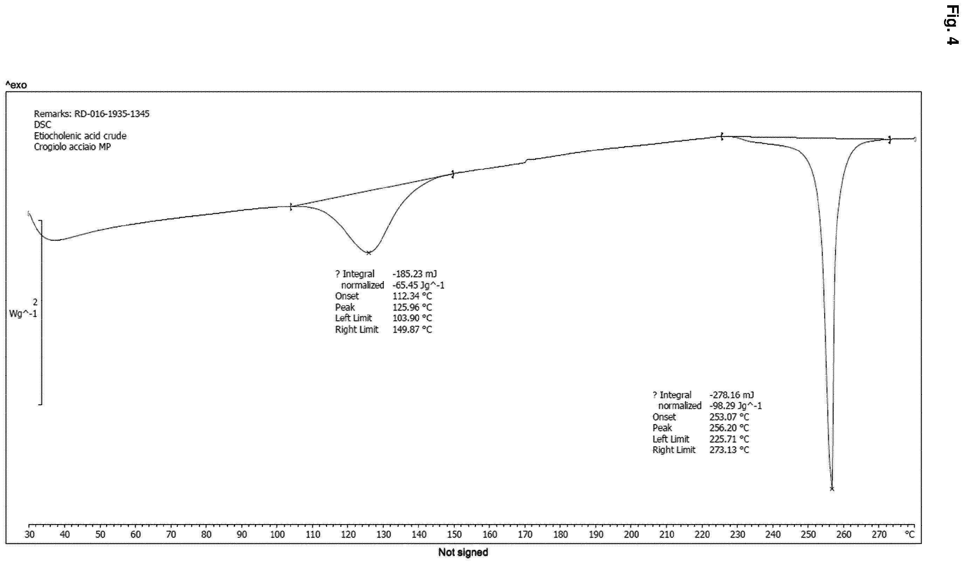

Etiocholenic acid toluene hemi-solvate samples were weighed into a medium pressure stainless steel crucible and heated at 10 °K/min from 30 % to 280 °C/min under nitrogen (80 mL/min). The results of these analyses are summarized in the following table 2 below. The DSCs performed are reported in figures 3 and 4.

**Table 2**

| Batch | Peak onset °C | Maximum Peak °C |
|---|---|---|
| RD-016- | 117.13 | 126.49 |
| 1935-1324 | | |
| RD-016- | 112.34 | 125.96 |
| 1935-1345 | | |

### Example 6 - XPRD Analysis

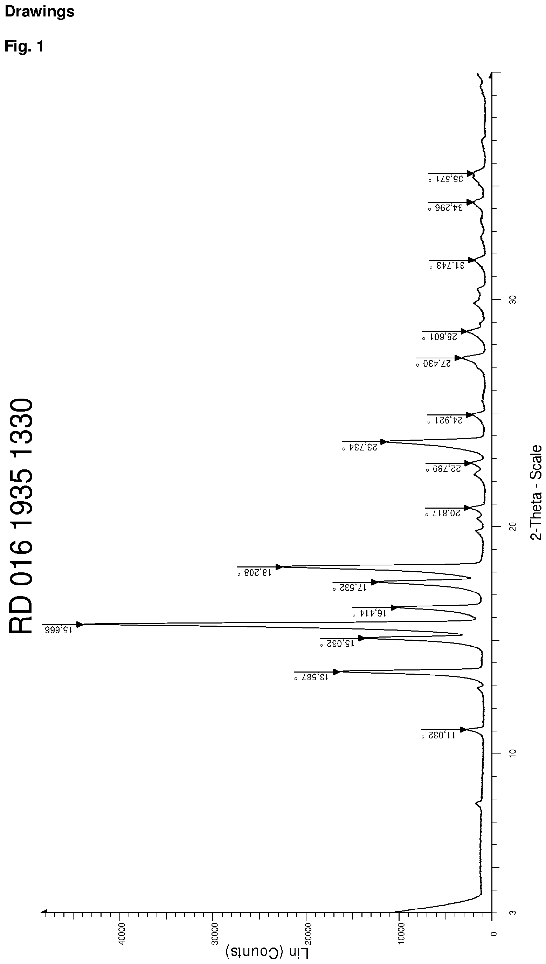

As far as the XPRD method is concerned, the instrument, the instrumental parameters and the other parameters used are reported in table 4 below. The XPRD diffractogram of FNT obtained is reported in figure 1.

| Method Instrument Parameters | |
|---|---|
| Instrument | Bruker AXS D8 Advance diffractometer |
| Generator | 40 kV/40 mA |
| Instrument Configu ration | Bragg-Brentano geometry (vertical Theta/Theta) |
| Tube | Ceramic X-ray Cu anode (LFF) tube |
| Radiation | Monocromated CuKa, g=1,5406 Å |
| Detector | LynxEye |
| Soller Slit | 2.5° |
| Divergent slit | 0.3° |
| Active length of detector | 3.6° |

| Diffracted beam - anti-scattering slit | 8 mm (Not Automated) |
|---|---|
| Diffracted beam filter | Ni Kb filter |
| Scan mode | Continuous |
| Phi rotation (spinner) | On (15rpm) |
| Sample holder | Bruker PMMA open specimen holder, sample reception Ø25mm |

| Acquisition Conditions | |
|---|---|
| Scan Range | 3-40° 2theta |
| Step size | 0.016° |
| Time/Step | 0.2 seconds |

### Example 7 - Comparison between the overall yields and the yields for each step (present invention vs prior art document WO 2006/002966)

| | WO 2006/002966 | Present process |
|---|---|---|
| Step 1 (crude etiocholenic acid) | 69% | 66% |
| | Example 1 | Example 1 |
| Step 2 (purified etiocholenic acid) | 84% | 94.8% |
| | Example 2 | Example 2 |
| OVERALL | 58% | 62,5% |

| | | |
|---|---|---|
| * Both examples starting from Progesterone | | |

### Example 8 - HPLC comparison between the crude etiocholenic acid profiles obtainable according to the invention and prior art document WO 2006/002966

The HPLC method carried out and related parameters was the following:
Phase A: water + H₃PO₄ 0,1%
Phase B: Acetonitrile
Gradient:

| Time [min] | A [%] | B [%] | Flow [mL/min] | Max pressure limit [bar] |
|---|---|---|---|---|
| 0.00 | 70.0 | 20.0 | 1.500 | 600.00 |
| 20.00 | 20.0 | 80.0 | --- | --- |
| 23.00 | 20.0 | 80.0 | --- | --- |
| 23.10 | 70.0 | 30.0 | --- | --- |
| 30.00 | 70.0 | 30.0 | --- | --- |

Injection: 5 uL
Column: Zorbax SB C8, 75 x 4.6 mm, 3.5 µm
Column temperature: 40 °C
Wavelength: 244 nm

The results of the HPLC analysis of crude etiocholenic acid showed:

| | WO 2006/002966 Example 1 | Present Invention Example 1 |
|---|---|---|
| Critical 1, RRT 0,72 | ? | Not detected |
| Critical 2, RRT 1,10 | ? | Always below 0.10% A/A |
| HPLC purity | 94% A/A | 99.26% A/A |

### Example 9 - Comparison HPLC purity purified etiocholenic acid

| WO 2006/002966 Example 2 | Present Invention Example 2 |
|---|---|
| 99% A/A | 99,4% A/A |

## Claims

1. Process for the purification of etiocholenic acid of formula (I) 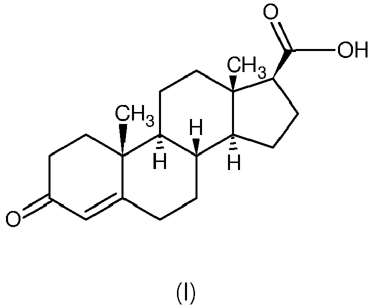 comprising the following steps:
a) preparing etiocholenic acid toluene hemi-solvate of formula (II) 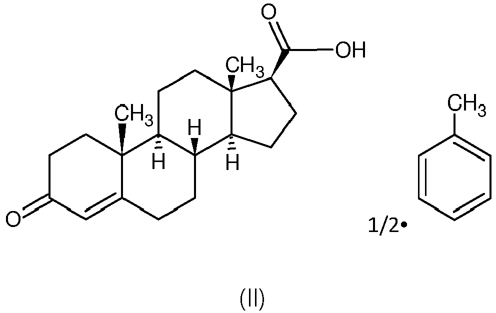 by treating crude etiocholenic acid with a solvent mixture comprising toluene;
b) converting the etiocholenic acid toluene hemi-solvate obtained in step a) to purified etiocholenic acid of formula (I).

2. Process according to claim 1, wherein the etiocholenic acid toluene hemi-solvate has a characteristic X-ray powder diffraction pattern with characteristic peak expressed in 2-Theta values (2θ) at 16.6 ± 0.2.

3. Process according to claim 1, wherein the etiocholenic acid toluene hemi-solvate has a characteristic X-ray powder diffraction pattern with characteristic peak expressed in 2-Theta values (2θ) at 16.6 ± 0.2 and/or at 14.365 ± 0.2 and/or at 8.2 ± 0.2.

4. Process according to any one of the preceding claims, wherein the etiocholenic acid toluene hemi-solvate has a DSC onset peak at a temperature among 110 °C and 120 °C and/or a peak at a temperature among 120 °C and 130 °C.

5. Process according to any one of the preceding claims, wherein the solvent mixture of step a) further comprises additional solvent selected from acetone and water.

6. Process according to any one of the preceding claims, wherein the solvent mixture comprises toluene, acetone and water.

7. Process according to any one of the preceding claims, wherein the solvent mixture comprises from 30 % to 60 % by volume of toluene.

8. Process according to any one of the preceding claims from 5 to 7, wherein the volume ratio between toluene : acetone : water is from 1.75 : 0.25 : 1.25 to 3.25 : 1.75 : 2.75.

9. Process according to any one of the preceding claims, wherein in the step b) of conversion, the etiocholenic acid toluene hemi-solvate is suspended in a solvent.

10. Process according to claim 9, wherein the solvent is acetone.

11. Process according to any one of the preceding claims from 9 to 10, wherein in the step b) of conversion, the etiocholenic acid toluene hemi-solvate is suspended in a solvent for a time from 0.5 hrs to 40 hrs.

12. Etiocholenic acid toluene hemi-solvate of formula (II):

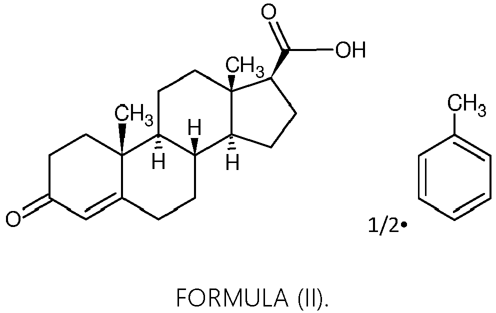

13. Etiocholenic acid hemi-toluene solvate according to claim 12 having the characteristics as defined in anyone of the preceding claims from 2 to 4.

14. Use of etiocholenic acid toluene hemi-solvate of formula (II): 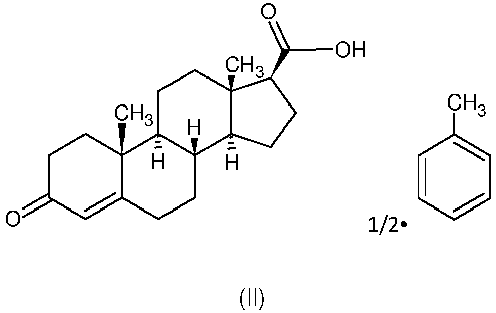 for preparing etiocholenic acid.
